# EUROPÄISCHE PATENTSCHRIFT

(11) **EP 0 825 826 B1**
(45) Veröffentlichungstag und Bekanntmachung des Hinweises auf die Patenterteilung: **23.10.2002**
(21) Anmeldenummer: 96916057.1
(22) Anmeldetag: 13.05.1996
(51) Int. Cl.: A61B 3/113, H04N 5/232, G02B 27/01

(54) **VERFAHREN UND VORRICHTUNG ZUM PARALLELEN ERFASSEN VON SEHINFORMATION**
PROCESS AND DEVICE FOR THE PARALLEL CAPTURE OF VISUAL INFORMATION
PROCEDE ET DISPOSITIF DE CAPTAGE EN PARALLELE D'INFORMATIONS PERCUES VISUELLEMENT

(30) Priorität: 15.05.1995 CH 141195
(43) Veröffentlichungstag der Anmeldung: 04.03.1998
(73) Patentinhaber: Leica Mikroskopie Systeme AG, 9435 Heerbrugg (CH)
(72) Erfinder: STÜTTLER, Herbert, M., A-6830 Rankweil (AT)
(74) Vertreter: Stamer, Harald
(86) Internationale Anmeldenummer: EP9602046
(87) Internationale Veröffentlichungsnummer: WO96036271

(56) Entgegenhaltungen:
- EP-A- 0 665 686
- WO-A-94/17636
- DE-A- 2 937 891
- DE-A- 4 337 098
- US-A- 3 170 979
- US-A- 4 582 403
- US-A- 5 341 181

## Beschreibung

Die Erfindung bezieht sich auf ein Verfahren nach Anspruch 1 und eine Vorrichtung nach Anspruch 9.

Bei manuellen Tätigkeiten, die äusserst genau ausgeführt werden müssen, hängt das Resultat stark von der exakten Beobachtung während der Tätigkeit ab. Dies gilt insbesondere bei chirurgischen Eingriffen am menschlichen oder tierischen Körper. Wenn die operierende Person das Gewebe im Bereich des Messers zuwenig genau beobachtet, besteht die Gefahr, dass eine wichtige Struktur - wie etwa ein Nervenbündel - durchtrennt wird.

In Systemen mit hohem Gefahrenpotential - beispielsweise in Atomkraftwerken - müssen Montage- und Kontrollarbeiten unter guter optischer Kontrolle sehr exakt durchgeführt werden. Ein exaktes Beobachten ist auch bei Manipulationen und bei Kontrollaufgaben in der Raumfahrt, der Fliegerei und in komplexen terrestrischen Systemen unumgänglich.

Der Wunsch, erfassbar zu machen was eine Person sieht, ist alt und konnte bis heute nicht optimal erfüllt werden. Der Versuch, die Bildinformation durch das Abtasten des Sehnervs zu erhalten, führt nicht zum Ziel, weil ein wesentlicher Teil der Signalbearbeitung und Bilderfassung erst im Hirn erfolgt.

Eine oder mehrere Video-Überwachungskameras, die den ganzen Bereich, der von einer Person kontrolliert werden soll, aufnehmen, zeigen lediglich auf, was erfassbar ist, nicht aber, was die beobachtende Person erfasst. Um ein mögliches Fehlverhalten der Person analysieren zu können, ist es sicher zweckmässig, wenn Überwachungskameras alles Erfassbare aufzeichnen. Zudem muss aber auch das Beobachtete erfasst werden.

Aus der Patentschrift US 4 395 731 ist eine Mikroskopbrille bekannt, die bei chirurgischen Eingriffen getragen werden kann. Der Operationsbereich wird von zwei vor den Augen angebrachten Videokameras überwacht. Den Augen werden die Bilder der Videokameras über direkt vor den Augen angeordnete Monitoren, bzw. Bildflächen zugeführt. Die Verwendung von Zoom-Optiken ermöglicht so die Vergrösserung von Ausschnitten des Operationsbereiches.

Die Augen bleiben immer auf die Bildquellen (z.B. ein CRT) gerichtet, was zu einer schnellen Ermüdung führen kann. Zudem kann nicht bestimmt werden, wie die Augen das ihnen zugeführte Bild erfassen. Es könnte sein, dass ein wichtiger Bildbereich in den Bildquellen nie so betrachtet wird, dass er auf jenen Bereich der Augenretina fällt, der das scharfe Sehen ermöglicht. Durch die Fokussierung und Ausrichtung der beiden Kameras ist eine feste Distanz vorgegeben, die zwischen den Kameras und dem Operationsbereich liegen muss, um eine scharfe und perspektivisch gute Ansicht zu erhalten. Ändert sich diese Distanz aufgrund von Kopfbewegungen, so sind die auf den Monitoren dargestellten Bilder unscharf und/oder können von den Augen nicht mehr so betrachtet werden, dass ein dreidimensionales Bild entsteht. Die manuelle Verstellmöglichkeit ist kaum geeignet, um die Ausrichtung der Kameras an kurzzeitige Distanzänderungen anzupassen. Ein weiterer Nachteil der Mikroskopbrille ist die Einschränkung des Gesichtsfeldes durch die Optiken der Kameras und durch die beschränkte Grösse der Monitore. Zudem sind Gesichtsbereichs-Änderungen mittels Augenbewegungen auf den Bildbereich der Kameras bzw. der Monitore eingeschränkt. Ein optisches System, das die Beobachtungen der arbeitenden oder kontrollierenden Person zu erfassen versucht, indem dieser Person nur die von Kameras erhaltenen Bilder zugeführt werden, schränkt die Beobachtungsfähigkeit dieser Person somit stark ein und zeigt nicht auf, welche Bereiche der zugeführten Bilder betrachtet werden.

Die erfindungsgemässe Aufgabe besteht nun darin, festzustellen, welche Bildinformationen von einer Person oder einem beliebigen lichtempfindlichen Sensorsystem tatsächlich erfasst werden.

Die erfindungsgemässe Lösung sieht ein aus der US 5 341 181 an sich bekanntes, parallel zu den Augen der Person oder parallel zu einem lichtsensiblen Sensorsystem wirkendes bildaufnehmendes Kontrollsystem vor, dessen Informationsaufnahme so wenigstens annähernd zur Deckung gebracht wird, dass wenigstens ein optischer Parameter der Augen oder des bildaufnehmenden ersten Systems erfasst wird und der entsprechende optische Parameter des Kontrollsystems in Abhängigkeit davon angepasst wird. Da das bekannte System aus dieser US-Patentschrift jedoch auf ein einziges, nicht stereoskopisch arbeitendes Kontrollsystem beschränkt ist, ist die optogeometrische Erfassung des gesehenen Bildes nur ungenau. Es wird demgegenüber erfindungsgemäss ein optisches System gemäß Anspruch 9 und ein Verfahren gemäß Anspruch 1 eingesetzt, das unter Beachtung der Stereobasis ( in der Regel des Augenabstandes) und der Ausrichtung der Fixierlinien der Augen zur Ermittlung des Fixierpunktes angewendet wird. Ist der Fixierpunkt bekannt, erfolgt die parallel erfasste Beobachtung der arbeitenden oder zu kontrollierenden Person wesentlich genauer als beim System gemäss der US 5 341 181. Es wird somit neu jede Augenstellung erfasst - vgl. Ansprüche 1 und 9. Weiterbildungen sind in den abhängigen Ansprüchen beschrieben bzw. gekennzeichnet. Vorzugsweise erfolgt die Bestimmung der optischen Achsen in einem Koordinatensystem, das relativ zum Kopf fest ist und sich somit mit dem Kopf bewegt. Da die Bewegungen jedes Auges durch sechs äussere Augenmuskeln erfolgt, kann eine Sensorvorrichtung vorgesehen werden, die die Reize dieser Muskeln erfasst und daraus die jeweilige Augenstellung bzw. die optischen Augenachsen ableitet.

Die Augenstellungen bzw. die Ausrichtungen der optischen Augenachsen, die in einem mit dem Kopf verbundenen Koordinatensystem bekannt sind, werden nun zur Steuerung der Ausrichtung mindestens einer, vorzugsweise aber zweier, über eine Halterung mit dem Kopf verbundener Kameras verwendet. Die mindestens eine Kamera ist dabei so mit dem Kopf verbunden, dass sie analog zu den Augen nebst den Bewegungen aufgrund der Kopfbewegungen auch Bewegungen relativ zum Kopf ausführen kann. Es versteht sich von selbst, dass zum Verstellen des zur Abbildung gelangenden Raumwinkelbereiches anstelle der Kamerabewegung auch eine verstellbare optische Abbildungsvorrichtung, insbesondere mit verstellbaren Spiegeln oder dergleichen, vorgesehen werden kann. Eine Anwendungsmöglichkeit ergibt sich auf den verschiedensten Gebieten, u.a. auch bei der Videotelefonie, bei der ein Gesprächspartner die Möglichkeit erhält, gewissermassen mit den Augen des Benutzers dieselben Dinge zu betrachten, ohne dass zusätzlich aufnahmetechnische Manipulationen nötig sind. In einer bevorzugten Ausführungsform ist auf beiden Seiten des Kopfes im Schläfenbereich im wesentlichen auf der Höhe der Augen eine Kamera mit einem CCD angeordnet. Jede Kamera ist jeweils dem nähergelegenen Auge zugeordnet. An einem auf den Kopf aufsetzbaren Halteteil ist eine die Kamerabewegungen ermöglichende Lagervorrichtung, insbesondere etwa eine kardanische Lagerung, und eine Betätigungsvorrichtung vorgesehen. Die Bewegung einer Kamera bzw. ihrer optischen Achse erfolgt beispielsweise durch die Bewegung eines im wesentlichen parallel zur Kameraachse verlaufenden und mit der Kamera verbundenen Stiftes. Dabei wird der Stift in einer Ebene quer zum Stift mittels zweier im wesentlichen senkrecht zueinander angeordneter Linearantriebe bewegt.

Da die Lage der Kameras etwas von der Lage der Augen verschoben ist, muss bei der Steuerung der Kameraausrichtung eine geometrische Korrektur berücksichtigt werden. Diese Korrektur soll gewährleisten, dass der Schnittpunkt der beiden Kameraachsen am gleichen Ort liegt, wie der Schnittpunkt der beiden optischen Augenachsen. Bei parallelen Augenachsen ist keine Korrektur nötig.

Um den von den Augen fixierten Punkt zu bestimmen, muss berücksichtigt werden, dass die Fixierlinie des Auges nicht mit der optischen Achse übereinstimmt. Bei der optischen Achse des Auges handelt es sich um die Achse, bezüglich welcher der optische Apparat des Auges (Cornea, Iris, Linse) im wesentlichen rotationssymmetrisch ist. Weil aber der empfindlichste Bereich der Retina, die Fovea centralis, etwas neben dieser Achse liegt, ist die Gesichtslinie bzw. Fixierlinie wenig gegen die optische Achse geneigt. Die Abweichung zwischen diesen beiden Linien kann als Standardabweichung berücksichtigt oder etwa bestimmt werden, indem bei vorgegebener Kopfausrichtung von den Augen ein Punkt mit bekannter Lage fixiert wird und dabei die Richtungen der optischen Augenachsen gemessen werden. Aufgrund der bekannten Kopfausrichtung bzw. der Lage der Augen und des vorgegebenen Fixierpunktes sind die Fixierlinien festgelegt, so dass die Abweichungen zwischen den optischen Augenachsen und den entsprechenden Fixierlinien bestimmbar sind.

Beim Berücksichtigen der unterschiedlichen Ausrichtung der optischen Achse und der Gesichtslinie eines Auges kann die mittels des Sensors bestimmte Richtung der optischen Achse aufgrund der bekannten Abweichung in die Richtung der Fixierlinie umgerechnet werden. Die Kameraachsen werden dann gemäss den Fixierlinienrichtungen auf den Fixierpunkt ausgerichtet, so dass der Fixierpunkt ins Zentrum des Bildbereiches zu liegen kommt. Werden die Kameras aber entsprechend den optischen Augenachsen ausgerichtet, so ist der Fixierpunkt etwas neben dem Bildzentrum. Die Lage des Fixierpunktes kann dann aufgrund der bekannten Abweichungen bei der Darstellung bzw. Auswertung der Kontrollbilder markiert bzw. berücksichtigt werden.

Falls das Kontroll-Beobachtungssystem keine dreidimensionale Bildinformation erfassen muss, genügt eine Ausführungsform mit lediglich einer Kamera, die vorzugsweise im Stirnbereich mittig über den beiden Augen angeordnet wird. Auch die Ausrichtung dieser Kamera soll auf den Schnittpunkt der Augenachsen, oder gegebenenfalls auf den Schnittpunkt der Gesichtslinien bzw. - bei fehlendem Schnittpunkt - parallel zu den Augenachsen erfolgen.

Anstelle des Ausrichtens mindestens einer Kamera kann etwa eine fest mit dem Kopf verbundene, vorzugsweise gerade nach vorne gerichtete, Kamera insbesondere mit Weitwinkeloptik eingesetzt werden, wobei die aus der Messung der Augenachsenrichtungen ableitbare Fixierlinie dann zum Bestimmen des betrachteten Bildbereichs verwendet wird. Durch das kontinuierliche Markieren des jeweiligen Fixierpunktes oder durch das Darstellen lediglich eines um den aktuellen Fixierpunkt angeordneten Bildbereiches kann erfasst werden, was die kontrollierte Person betrachtet.

Aus dem Bereich der Foto- und Videokameras sind Verfahren und Vorrichtungen zum Bestimmen der Ausrichtung der optischen Augenachse bekannt. Aufgrund der bestimmten Achsenlage erfolgt etwa die automatische Fokussierung auf den betrachteten Bildbereich. Zur Achsenbestimmung wird das Auge mit Infrarotlicht, das vom Auge nicht wahrgenommen wird, beleuchtet und das reflektierte Infrarotlicht bzw. das Augenbild wird auf eine Bildsensorebene abgebildet. Das Augenbild setzt sich im wesentlichen aus Reflexionen an der Hornhaut (Cornea) und der Iris zusammen. Da der grösste Anteil des durch die Pupille gelangenden Infrarotlichtes nicht zurückgeworfen wird, kann mit dem Bildsensor und mit einer damit verbundenen Bildauswertungseinheit ein Unterschied im Randbereich zwischen Pupille und Iris ermittelt werden. Gegebenenfalls wird auch die von der Augenrichtung abhängige Form der Iris bestimmt und das Zentrum der Pupille als Schnittpunkt der zwei Hauptachsen, der im Bild ellipsen- oder gegebenenfalls kreisförmigen äusseren Iris-Berandung festgelegt. Die optische Augenachse ist dann die Linie, die durch das Zentrum der Pupille und durch ein vorgängig zu bestimmendes Augendrehzentrum führt.

Zum Bestimmen des Augendrehzentrums wird etwa bei fester bekannter Kopfausrichtung und Kopflage mindestens für zwei bekannte, nacheinander von den Augen fixierte Fixierpunkte eine Fixierlinien-Bestimmung durchgeführt. Die Fixierpunkte sind vorzugsweise zwei fest mit der Halterung für die Kameras und die Sensoren zur Bestimmung der Pupillenlage verbindbare Markierpunkte, so dass die Bestimmung unabhängig von der Kopfausrichtung erfolgen kann. Das Augendrehzentrum liegt dann im wesentlichen beim Schnittpunkt der durch die Fixierpunkte gelegten Fixierlinien. Da das Augendrehzentrum auf der optischen Augenachse liegt, muss zur Erhöhung der Bestimmungsgenauigkeit aufgrund der Abweichung zwischen Fixierlinie und optischer Augenachse, wie oben beschrieben, eine Korrektur gemacht werden. Der Abstand zwischen den Drehzentren der beiden Augen entspricht dem Augenabstand. Wenn die beiden Augen im wesentlichen gerade und horizontal nach vorne ins Unendliche blicken, werden die beiden Iris je kreisförmig auf die beiden Bildsensorebenen abgebildet. Der Abstand zwischen den Pupillenzentren entspricht ebenfalls dem Augenabstand und die Pupillenzentren liegen je in einem Auslenkungsnullpunkt. Um die Augen unabhängig von der Kopfausrichtung parallel gerade nach vorne auszurichten, wird gegebenenfalls gerade vor jedem Auge ein mit der Halterung verbundener Markierpunkt angeordnet. Die Augen sind dann parallel nach vome ausgerichtet, wenn die beiden je einem Auge zugeordneten Markierpunkte in den Bildern beider Augen aufeinander liegen.

Durch die Bestimmung des Randbereiches zwischen Pupille und Iris ist auch die eingestellte Blende bekannt. Diese Blende soll gegebenenfalls auch als gemessener Parameter zur Steuerung der Blende des Kontrollsystems verwendet werden und so die Lichtverhältnisse analog zu jenen im Auge variieren. Dies ist dort von Vorteil, wo man eher am tatsächlich durch das Auge wahrgenommenen Bild interessiert ist als an einem möglicherweise besseren, das durch die Kamera aufgenommen wird (z.B. bei medizinischen Anwendungen). Um im Kontrollsystem für die Bildaufnahme optimale Lichtverhältnisse zu haben, wird die Blende automatisch verstellt, dabei geht jedoch gegebenenfalls wahlweise der tatsächliche Helligkeits- und Schärfeneindruck des Anwenders verloren.

Bei den Augenachsen-Bestimmungsverfahren gemäss dem Stande der Technik wird das vom Auge zurückgeworfene Infrarotlicht durch einen teildurchlässigen Infrarot-Spiegel (dichroitischer Spiegel), dessen Reflexionsrate für Infrarot sehr hoch und für sichtbares Licht sehr klein ist, seitlich aus dem Strahlengang der Kamera gegen den Bildsensor ausgelenkt. Dadurch wird gewährleistet, dass die Sicht auf die Bildebene der Kamera ungestört ist. Bei der erfindungsgemässen Lösung schaut das Auge hingegen weder durch ein Okular noch auf eine bilddarstellende Fläche, sondern direkt auf die Objekte. Um die Achsbestimmung durchzuführen, muss aber vor dem Auge mindestens ein Infrarot-Spiegel vorgesehen sein, der zumindest das Infrarotbild des Auges auf einen Bildsensor seitlich am Rand des Gesichtsfeldes umlenkt. Gegebenenfalls wird aber auch das infrarote Beleuchtungslicht von mindestens einer seitlichen Quelle über einen Spiegel dem Auge zugeführt.

Bei den bekannten Anwendungen der Augenachsenbestimmung ist das Auge jeweils nahe am Okular der Sucheranordnung, so dass das durch die Infrarotmessung bestimmte Augenbild nicht oder nur wenig durch Reflexionen des Umgebungslichtes beeinträchtigt wird. Bei der erfindungsgemässen Anordnung ist das Auge im wesentlichen ungeschützt dem Umgebungslicht ausgesetzt. Um eine Störung des Augenbildes und seiner Auswertung durch Umgebungslicht zu verhindern, sieht die DE 43 37 098 A eine Betriebsweise vor, bei der die Bildinformation auf der Basis des Unterschiedes zwischen Phasen mit Infrarotbeleuchtung und Umgebungslicht und Phasen nur mit Umgebungslicht ausgewertet wird. Aus der EP 602 895 A ist ein äusserst kompakter Achsbestimmungssensor bekannt.

Da das Auge in der erfindungsgemässen Anordnung im wesentlichen uneingeschränkt dem Umgebungslicht ausgesetzt ist, ist es jedoch gut beleuchtet und erlaubt gegebenenfalls die Bestimmung der Pupillenlage mit einem Bildsensor (CCD), der für Licht im sichtbaren Bereich empfindlich ist. Bei der Achsenbestimmung kann somit auf eine Beleuchtung mit Infrarot verzichtet werden.

Nebst der Ausrichtung der Augen ist die Akkommodation durch die Einstellung der Brechkraft der Linse ein weiterer wichtiger Parameter des Beobachtungsvorganges. In einer ersten Näherung kann davon ausgegangen werden, dass die Brechkraft der beiden Augen immer auf den Fixierpunkt eingestellt ist. Entsprechend sollen die Optiken der Kameras so gesteuert werden, dass der Fixierpunkt, dessen Abstand aufgrund des Abstandes zwischen den beiden Augen und den Ausrichtungen der Fixierlinien (Triangulation) bestimmbar ist, scharf gestellt wird. Die Bestimmung des Fixierpunkt-Abstandes mittels Triangulation ist zumindest im Nahbereich genügend genau. Gegebenenfalls können die Optiken der Kameras auch mittels Autofokus im Bereich des Fixierpunktes scharfgestellt werden.

Zur Bestimmung der Brechkraft des Auges werden Augenrefraktometer eingesetzt, die im wesentlichen die sphärische Dioptrie, den Astigmatismus und die Astigmatismusachse messen. Da sich der Astigmatismus und die dazugehörige Achse zumindest in erster Näherung während eines Beobachtungsvorganges nicht ändern, muss lediglich die sphärische Dioptrie bestimmt werden, um die Brechkraft des Kontrollsystems mittels gemessener Parameter zu steuem. Die Messung dieses Parameters wird dadurch erschwert, dass er bei beliebiger Ausrichtung des Auges bestimmt werden muss.

Die Änderung der Linsenbrechkraft geht mit Formänderungen der Linse einher, die zumindest in bestimmten Richtungen oder Schnittebenen gegebenenfalls mittels Ultraschallechographie bestimmbar sind. Zum Ausmessen des Auges entlang der optischen Achse wird ein Ultraschallsignal über eine Vorlaufstrecke aus Wasser an das Auge angekoppelt. Die Linsendicke ergibt sich durch Laufzeitdifferenzen der Echos an den beiden Linsenberandungen. Nebst den reinen Laufzeitmessungen entlang einer Achse werden auch beim Auge bildgebende Ultraschall-Messverfahren verwendet, die Schnittbilder durch das Auge darstellen. Bei der erfindungsgemässen Vorrichtung muss das Auge eine im wesentlichen ungestörte Sicht nach vorne haben. Eine gegebenenfalls vorgesehene Ultraschallankoppelung muss daher von der Augenseite her erfolgen. Zur Berechnung der aktuellen Brechkraft muss dabei die in mindestens einem Schnittbild erscheinende Gestalt der Linse zusammen mit der aktuellen Augenachse verwendet werden.

Eine Ultraschallmessung hat gegenüber den Messungen mit Licht den Vorteil, dass das Innere des Auges und somit auch die Linse nicht nur im wesentlichen entlang der optischen Achse, also in einer Richtung, in welcher das Auge lichtdurchlässig ist, analysiert werden kann. Dafür sind die Messgenauigkeiten kleiner und die nötigen Umrechnungen aufwendiger.

Bei der bevorzugten optischen Messung der Brechzahl des Auges ist es wichtig, eine geeignete Einstellung des Refraktometers zum zu untersuchenden Auge herzustellen und den Abstand zwischen beiden genau zu justieren. Zudem ist es wesentlich, die nachteiligen Auswirkungen des Augenzwinkerns auf die Messung auszuschliessen. Aus der DE 29 37 891 A ist ein Refraktometer bekannt, das in einem ersten Schritt optimal auf das auf ein Fixationsobjekt gerichtete, zu untersuchende Auge eingestellt wird. In einem weiteren Schritt wird eine verstellbare Testbildstruktur - vorzugsweise definiert ausgerichtete Lichtstrahlen - durch die Linse auf die Netzhaut abgebildet. Das reflektierte, aus dem Auge austretende Licht des Bildes auf der Netzhaut und dessen Änderung beim Verstellen des Testbildes wird analysiert, um die mittlere Dioptrie (sphärisch), den Astigmatismus (zylindrisch) und die Astigmatismusachse (Zylinderachse) zu bestimmen.

Bei einem bewegbaren Auge, dessen optische Achse aufgrund einer Messung bekannt ist, muss ein Infrarot-Testbild jeweils entlang der aktuellen Augenachse auf die Netzhaut abgebildet werden können. Dazu wird beispielsweise von einem Leuchtdiodenarray oder einer von hinten durchleuchteten Flüssigkristall-Bildfläche ein Testbild erzeugt, das von einem optischen System umgelenkt, gegebenenfalls fokussiert und durch die Pupille ins Auge geführt wird. Die Abbildung auf der Netzhaut wird wiederum mittels Umlenkung und etwa Fokussierung einer Bildaufnahmevorrichtung, bzw. Infrarotsensoren zugeführt.

Indem die Testbilder von verschiedenen Bereichen des Arrays bzw. der Bildfläche ausgehen, kann mit einer geeigneten Optik erreicht werden, dass die Bilder entlang der jeweils aktuellen optischen Achse - gegebenenfalls im wesentlichen fokussiert - auf das Auge geführt werden können. Mittels Variationen der Testbilder kann das Abbildungsverhalten der Augenoptik bestimmt werden. Es versteht sich von selbst, dass die im freien Gesichtsfeld des Auges angeordneten optischen Elemente, insbesondere die Umlenkelemente bzw. dichroitische Spiegel, deren Aufbau z.B. aus der japanischen Patentanmeldung JP 88865/1978 bekannt ist, für Licht im sichtbaren Bereich ungehindert passierbar sein sollen. Zur Strahlumlenkung sind nebst flacher Spiegel auch gekrümmte Spiegel verwendbar. Mittels zweier viertelkugelförmiger, konkaver, je von zwei entgegengesetzten Seiten beleuchtbarer Spiegel wäre das Auge im wesentlichen aus jeder möglichen Augenachsenrichtung beleuchtbar.

Bei dem oben beschriebenen Messsystem mit einer Sender- und einer Empfängermatrix kann anstelle des Übertragens von Testbildern auch ein sequentielles Senden und paralleles Empfangen vorgesehen werden. Dabei wird etwa zumindest ein Teil der Leuchtdioden des Diodenarrays hintereinander ein- und ausgeschaltet und die rückgestreuten Bilder werden vorzugsweise zusammen mit der ebenfalls bestimmten Achsenlage zur Bestimmung mindestens eines Akkommodationsparameters verwendet. Mit einem System mit einer Sender- und einer Empfängermatrix kann insbesondere auch die Pupillenlage und somit die Achsenlage bestimmt werden.

Anstelle eines Leuchtdiodenarrays kann gegebenenfalls auch mindestens ein richtungs- und insbesondere auch lageverstellbarer Infrarot-Diodenlaser eingesetzt werden. Der Laserstrahl müsste durch ein optisches System und über ein dichroitisches Einblendelement im wesentlichen entlang der aktuellen Augenachse zur Abbildung auf die Netzhaut ausrichtbar sein. Das aus dem Gesichtsfeld ausgelenkte und gegebenenfalls durch eine verstellbare Optik geführte Rückstreubild der Netzhaut müsste mindestens einem Infrarotsensor, gegebenenfalls aber einem Sensorarray, zuführbar sein.

Durch das kontinuierliche Bestimmen der optischen Augenachsen, der Pupillenweite und der Akkommodation während eines Sehvorganges wird das parallele Erfassen von Bilddaten mittels Kameras und anhand dieses Bildmaterials eine dynamische Sehanalyse ermöglicht. Akkommodationsfehler können durch den Vergleich, der Lage des Fixierpunktes mit der gemessenen Dioptrie bzw. Akkommodations-Distanz erkannt werden. Bei der Vorgabe von dreidimensional bewegten Fixierpunkten kann kontrolliert werden, wie die Augen diesen Punkten folgen. Dynamische Sehanalysen können auch beim Lesen von Text oder Musiknoten, insbesondere auch beim Überwachen von Instrumenten-Anzeigen, Augenführungsfehler aufzeigen. Nebst Kontroll-, Leit- und Diagnoseaufgaben sind auch therapeutische Anwendungen möglich. Übungen im Grenzbereich des Auftretens erkannter Sehfehler, insbesondere etwa Akkommodationsgrenzen oder Probleme beim exakten Einstellen beider Augen auf einen einzigen Punkt (Fusionsprobleme), können unter interaktiver Kontrolle das Sehverhalten verbessern. Im Musikunterricht kann sowohl gutes wie schlechtes Notenleseverhalten aufgezeichnet und anhand der Aufzeichnungen analysiert werden. Die Optimierung des Sehverhaltens kann auch in Sportarten, wie etwa Tennis, Schiessen und bei Spielen mit Torhütern von Bedeutung sein. Personen in einem Kontrollzentrum können so über Funk einem Anwender unmittelbare Anweisungen geben, z.B. bestimmte Gegenstände zu fixieren.

Ein weiteres Anwendungsgebiet des erfindungsgemässen Erfassens mit einem bildaufnehmenden Kontrollsystem ist das blickgesteuerte Ansteuern von Menuefeldern auf einem Bildschirm. Diese Möglichkeit ist insbesondere bei Operationen, bei denen die operierende Person die Operation am Bildschirm verfolgt, vorteilhaft. Die Menuefelder werden vorzugsweise durch Symbole gekennzeichnet, die zu Erkennungszwecken in einem Bildverarbeitungssystem gespeichert sind. Die Bildausschnitte des Kontrollsystems im Bereich des Fixierpunktes sind mittels Bildverarbeitung mit den gespeicherten Symbolen vergleichbar. Dazu ist sowohl die Bildschirminformation als auch die Bildinformation des Kontrollsystems der Bildverarbeitung zugänglich. Wenn nun die kontrollierten Anwenderaugen ein Menuefeld bzw. das dazugehörige Symbol über eine vorgegebene Zeit fixieren, oder über ein Schaltorgan, das etwa mechanisch oder akustisch betätigbar ist, den Erkennungsvorgang auslösen, wird das betrachtete Symbol - wenn möglich - einem entsprechenden gespeicherten Symbol zugeordnet. Bei einer erfolgreichen Zuordnung werden gegebenenfalls weitere Menuefelder auf dem Bildschirm dargestellt oder aber ein Befehl wird - gegebenenfalls nach einer Bestätigung - ausgeführt.

Das blickgesteuerte Ansteuern von Menuefeldern durch ein Okular ist beispielsweise aus dem Bereich von Videokameras bekannt. Dort muss lediglich eine Augenachse bestimmt werden, um den betrachteten Bildbereich erkennen bzw. zuordnen zu können. Bei frei beweglichem Kopf und Augen müsste die Nullpunktslage und die Orientierung des kopffesten Koordinatensystems, sowie in Koordinaten desselben die Augenachse bzw. Fixierlinie bekannt sein, um eine Fixierlinie richtig im dreidimensionalen Raum anordnen und gegebenenfalls mit der Bildschirmebene schneiden zu können. Nebst der Bestimmung der Augenachsen müssten die Bewegungen des Kopfes telemetrisch überwacht werden. Um eine Fixierlinie im dreidimensionalen Raum anzuordnen, ist der Aufwand gross und zudem besteht zumindest bei kleinen Menuefeldern die Gefahr, dass nicht das richtige Feld bestimmt wird. Das Vergleichen von betrachteten Symbolen mit vorgegebenen Symbolen dürfte somit in den meisten Fällen einfacher und effizienter sein.

Eine weitere Anwendung, die im wesentlichen ähnlich ist zum oben beschriebenen blickgesteuerten Ansteuern von Menuefeldern, ist das Erkennen von kontrollierten Instrumenten-Anzeigen. Beim Überwachen von Instrumenten ist es wichtig, dass einzelne Anzeigen in vorgegebenen Zeitintervallen mindestens einmal von der kontrollierenden Person abgelesen werden. Das Bildmaterial des Kontrollsystems muss diese Anzeigen somit in den vorgegebenen Zeitintervallen mindestens einmal im Fixierbereich darstellen. Dies kann mittels Bildverarbeitung kontrolliert werden.

Ein weiteres Anwendungsgebiet des erfindungsgemässen Erfassens mit einem bildaufnehmenden Kontrollsystem ist die vom Blick gesteuerte Cursor-Führung auf einem betrachteten Bildschirm. Anstatt - wie oben beschrieben - Bildausschnitte des Kontrollsystems im Bereich des Fixierpunktes mittels Bildverarbeitung gespeicherten Symbolen zuzuordnen, können auf dem betrachteten Bildschirm Koordinatenmarken vorgesehen werden, die mittels Bildverarbeitung im erfassten Bild bestimmbar sind. Die Lage des Fixierpunktes bzw. Bildzentrums des von den entsprechend der Augenausrichtung ausgerichteten Kameras aufgenommenen Bildes des Bildschirmes, kann dann in Bildschirmkoordinaten umgerechnet werden. Die Bildschirmkoordinaten des Fixierpunktes der Augen und somit der Kameras werden an den Bildschirm bzw. seine Steuerung übertragen, so dass der Fixierpunkt gemäss seiner Koordinaten auf dem betrachteten Bildschirm in der Form eines Cursors dargestellt werden kann.

Die blickgesteuerte Cursor-Führung auf einem betrachteten Bildschirm hat sehr viele Verwendungsmöglichkeiten. Bei Operationen, bei denen die operierende Person die Operation am Bildschirm verfolgt, kann der Blickcursor der operierenden Person für die assistierenden Personen eine Orientierungshilfe sein, die das Verstehen des Operationsvorganges bzw. Erklärungen dazu und das Ausführen von Anweisungen erleichtert. Mit dem blickgesteuerten Cursor können Menuefelder angesteuert, Zoombereiche lokalisiert, oder sogar Bewegungen des das Bild erzeugenden Aufnahmesystems und gegebenenfalls von Operations-Hilfsgeräten gesteuert werden.

Es versteht sich von selbst, dass die blickgesteuerte Cursor-Führung grundsätzlich alle bekannten Cursor-Führungen - also auch die verbreitete Maus-Führung - ersetzen kann. Diese Ersatzmöglichkeit ist besonders vorteilhaft für körperlich stark behinderte Personen. Selbst wenn nur die Augen bewegt werden können, ist mit der blickgesteuerten Cursor-Führung eine vielseitige und effiziente Computerbenützung möglich.

Beim Auge ist das Bildauflösungsvermögen beim auf die Fovea centralis abgebildeten Bildbereich maximal. Von diesem Bereich aus nimmt die Auflösung in alle Richtungen stark ab. Im Bereich des blinden Flecks ist sie sogar verschwindend klein. Das von einer Kamera erfasste Bild hat normalerweise über den ganzen Bildbereich im wesentlichen die gleiche Auflösung. Um nun die vom Beobachter erhaltene Bildinformation zu erzeugen, kann die Bildinformation des Kontrollsystems mittels Bildverarbeitung unter Berücksichtigung des realen Bildauflösungsvermögens des Auges vom zentralen Bildbereich nach aussen reduziert werden. Gegebenenfalls wird auch bereits der Kamera des Kontrollsystems eine das gewünschte Auflösungsvermögen erzeugende Weichzeichnerlinse vorgeschaltet. Wird die Auflösungsreduktion mittels Bildverarbeitung erst bei der Analyse des aufgezeichneten Bildmaterials des Kontrollsystems durchgeführt, so besteht die Möglichkeit, die optimale Beobachtung mit einer realen zu vergleichen, insbesondere zu bestimmen, welche Informationen beim Auge untergehen.

Derartige Vergleiche dürften beim Beurteilen chirurgischer Haftpflichtfälle von Bedeutung sein, da Fahrlässigkeit meist nur dann vorliegt, wenn etwa vor einem Fehlschnitt, dessen Gefährlichkeit erkennbar war, oder wenn ein Fehler von der operierenden Person gesehen wurde, ohne darauf zu reagieren. Ein anderes erfindungsgemässes bildaufzeichnendes Überwachungssystem, das Bilder aus einer von der operierenden Person abweichenden Perspektive und ohne die Einschränkungen des menschlichen Auges aufzeichnet, macht gegebenenfalls Bildinformationen zugänglich, die für die operierende Person nicht zugänglich waren.

Es versteht sich von selbst, dass nebst dem Verändern der Bildauflösung auch andere bildverändernde Bearbeitungsschritte, wie etwa das Verstärken von Konturlinien oder das Einfügen von Masken, vorgesehen werden können. Dabei kann zur Bildaufbereitung auch Bildmaterial von anderen bildgebenden Verfahren verwendet werden. Bei starken kurzzeitigen Augenbewegungen müssen gegebenenfalls für die Kamerabewegungen Dämpfungsverfahren und für die aufgezeichneten Bilder Filterverfahren vorgesehen werden.

Analog zur Verarbeitung des von der Retina aufgenommenen Bildes im Gehirn muss das Bildmaterial gegebenenfalls bearbeitet werden. Kontraste sollen verstärkt, Unschärfen ausgeglichen, periphere Informationen gefiltert und Bewegungen des Kopfes und der Aussenwelt ausgeglichen werden, so dass ein ruhigstehendes Bild entsteht. Nebst dem Bildmaterial werden gegebenenfalls auch die gemessenen Parameter gefiltert und/oder bearbeitet, so dass eine optimale Steuerung des Kontrollsystems und somit eine optimale Bildaufnahme möglich wird.

Ebenfalls durch bildverändernde Bearbeitungsschritte kann beispielsweise ein Verständnis für ein anderes Sehverhalten bzw. Sehvermögen erzielt werden. Das Bildmaterial des Kontrollsystems wird dazu mittels Bildverarbeitung dem anderen Sehen, beispielsweise dem Sehen bei Netzhautablösung oder etwa dem Sehen von Tieren, deren Augen andere Charakteristiken haben, angepasst.

Nebst dem Messen von Augenparametern und der Bildaufnahme mittels eines Kontrollsystems kann gegebenenfalls auch vorgesehen werden, dass mindestens einem Auge über ein teildurchlässiges optisches Umlenkelement Bilder zugeführt werden können. Die einblendbaren Bilder können dabei etwa aus dem Kontrollsystem stammen und den gegebenenfalls wegen eines Fehlverhaltens schlecht sehenden Augen aufzeigen, welches Bild bei optimalem Sehverhalten zu sehen wäre. Entsprechend kann auch umgekehrt einer normalsichtigen Person das Bildmaterial, das einer fehlsichtigen Person zugänglich ist, eingeblendet werden.

Bei chirurgischen Eingriffen ist es zuweilen zweckmässig, wenn der operierenden Person Bilder von anderen bildgebenden Verfahren, wie etwa Röntgenbilder, Ultraschallbilder, Computertomogramme und Kernspinresonanzbilder - insbesondere dem einsehbaren Operationsbereich überlagert - zuführbar sind. Dabei wird gegebenenfalls vom Auge aus gesehen hinter dem die Bilder zuführenden Umlenkelement ein elektronisch schaltbares Schliesselement vorgesehen, das z.B. aufgrund von Flüssigkristalländerungen von einem transmissiven in einen reflektiven Zustand übergeht. Dadurch ist es möglich, die zugeführten Bilder dem aktuellen Hintergrund überlagert, oder ohne Hintergrund zu betrachten.

Das Kontrollsystem umfasst gegebenenfalls eine Zoomoptik, so dass bei Bedarf ein gezoomter Ausschnitt im Bereich des von den Augen fixierten Punktes erfasst wird. Durch das zeitweise Einblenden des Zoombildes kann beispielsweise eine operierende Person in heiklen Operationsphasen von der direkten Beobachtung auf eine Beobachtung eines vergrösserten Teilbereiches umstellen (vergleichbar der bekannten Mikroskopbrille). Die Ausrichtung der Augen wird während des Betrachtens des Zoombildes vorzugsweise nicht mehr zum Bewegen der optischen Achsen der Kameras des Kontrollsystems verwendet, sondern etwa - wie bei Operationsmikroskopen gemäss der Patentanmeldung CH 3217/94-9 - zum menuegesteuerten Verstellen von Parametern des Kontrollsystems benützt. Gegebenenfalls werden Schwankungen der Distanz zwischen den Kameras und dem beobachteten Bereich automatisch mittels Autofokus und insbesondere durch kleine Ausrichtungsänderungen der Kameras kompensiert.

Nebst dem Erfassen und gegebenenfalls Einblenden eines Zoombildes kann das Kontrollsystem auch Bilder in einem vom sichtbaren Bereich abweichenden Wellenlängenbereich, insbesondere im Infrarotbereich, Bilder mit einem vergrösserten Gesichtsfeld und/oder mit einer veränderten Stereobasis erfassen und gegebenenfalls den Augen zuführen.

Beim ersten bildaufnehmenden System handelt es sich vorzugsweise um mindestens ein Auge, insbesondere um ein menschliches Auge oder tierisches Auge. Grundsätzlich können aber alle lichtsensiblen Sensorsysteme, gegebenenfalls sogar Einzelsensoren, Systeme mit mehreren Sensoren, vorzugsweise aber Sensorarrays und Bildaufnahmevorrichtungen mit einem bildaufnehmenden Kontrollsystem, kontrolliert werden. Vorzugsweise wird zumindest eine Richtungsachse des Sensorsystems erfasst und die mindestens eine Kamera wird in Abhängigkeit von dieser Richtung ausgerichtet. Der Einsatz eines bildaufnehmenden Kontrollsystems zur Kontrolle eines lichtsensiblen ersten Systems ermöglicht die Kontrolle des situationsabhängigen Verhaltens des ersten Systems.

Es versteht sich von selbst, dass die Bildinformation des Kontrollsystems und gegebenenfalls die am ersten bildaufnehmenden System gemessenen Parameter aufgezeichnet werden können.

Analog zu den Verfahrensansprüchen sind weitere Vorrichtungsansprüche denkbar, die gegenständliche Merkmale aufweisen, mit denen die entsprechenden Verfahrensschritte durchgeführt werden können.

Die Zeichnungen erläutern die Erfindung an Hand schematisch dargestellter Ausführungsformen, auf welche die Erfindung aber nicht eingeschränkt ist.
- Fig.1: Frontansicht eines Kopfes mit zwei seitlich der Augen angeordneten Videokameras und zwei über den Augen angeordneten Sensoren zur Bestimmung der Augenachsen;
- Fig.2: Seitenansicht eines Kopfes mit Videokamera, Augenachsensensor und Umlenkspiegel;
- Fig.3: Seitenansicht eines Kopfes mit Videokamera, Augenachsensensor, Umlenkspiegel, Bildeinblendevorrichtung und schaltbarem Schliesselement;
- Fig.4: Ansicht eines Systems mit einer von Hand ausrichtbaren ersten Kamera, einer Kontrollkamera und einer Mess-, Steuer- und Bildaufnahmevorrichtung.

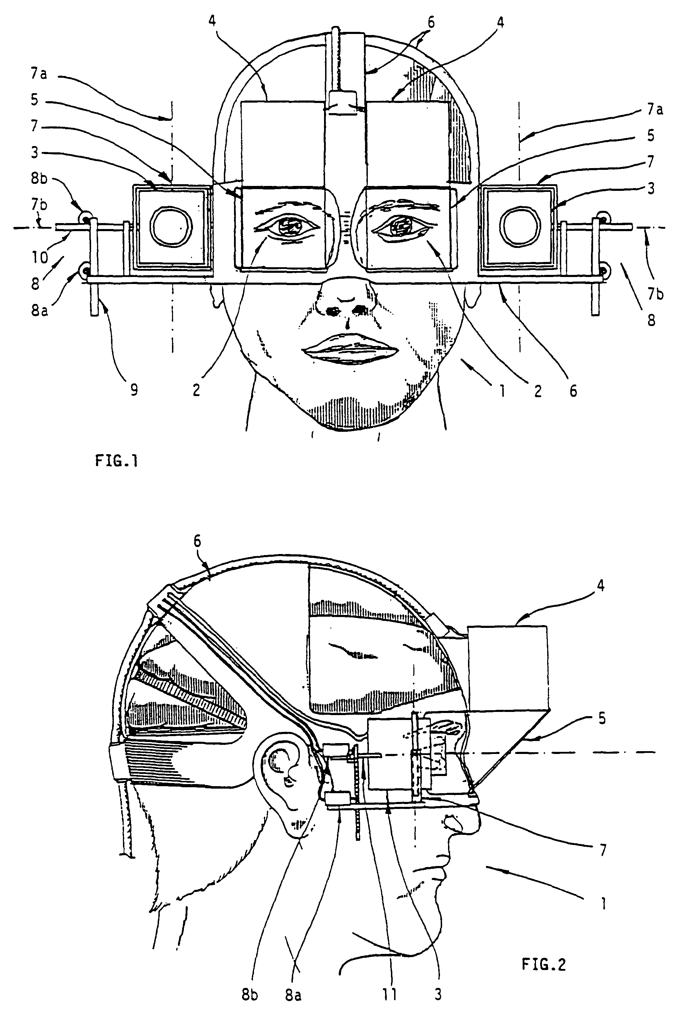

Fig. 1 zeigt einen Kopf 1 mit zwei seitlich der Augen 2 angeordneten Videokameras 3 und zwei über den Augen 2 angeordneten Messvorrichtungen, die etwa Augen-Ausrichtungs-Sensoren 4 umfassen, aus deren Messung bzw. Bildaufnahme die Lage des Pupillenzentrums ableitbar ist. Um die Augenbilder den Ausrichtungs-Sensoren 4 und gegebenenfalls Licht, insbesondere Infrarot, von den Ausrichtungs-Sensoren 4 den Augen 2 zuzuführen, sind vor beiden Augen erste teildurchlässige Umlenkelemente 5 bzw. Spiegel - vorzugsweise dichroitische Spiegel - vorgesehen. Gegebenenfalls umfasst die Messvorrichtung auch eine Sensoranordnung und eine Auswertungseinheit zur Bestimmung der Brechkraft jedes Auges. Die Sensoren 4, die teildurchlässigen Spiegel 5 und die Kameras 3 sind an einer Halterung 6 befestigt, die auf den Kopf 1 aufsetzbar ist.

Als Lagerungsvorrichtungen für die Kameras 3 sind vorzugsweise kardanische Lager 7 mit zueinander im wesentlichen normal stehenden ersten und zweiten Bewegungsachsen 7a und 7b vorgesehen. Um die Kameras 3 entsprechend der gemessenen Augenausrichtungs-Parameter in gewünschte Richtungen relativ zu einem mit dem Kopf bzw. der Halterung 6 verbundenen Koordinatensystem auszurichten, ist vorzugsweise jeder Kamera ein zweidimensionales Stellsystem 8 zugeordnet. Die Stellsysteme 8 betätigen etwa einen nach hinten stehenden Kamerafortsatz mittels zweier - eines ersten und eines zweiten - z.B. entlang der Achsen 7a und 7b wirkender, Linearantriebe 8a und 8b. Dabei ist der erste Antrieb 8a mit der Halterung 6 verbunden und bewegt eine erste Stange 9, an deren Endbereich der zweite Antrieb 8b befestigt ist. Der zweite Antrieb 8b ist über eine zweite Stange 10 mit dem Kamerafortsatz verbunden.

Fig.2 zeigt den Kopf 1 mit der Halterung 6 von der Seite, so dass auch der Kamerafortsatz 11 erkennbar ist. Aus der Seitenansicht geht auch die Halterung und Ausrichtung der teildurchlässigen Spiegel 5 gut hervor. Die von den Sensoren 4, den Stellsystemen 8 und den Kameras 3 ausgehenden Leitungen sind vorzugsweise mit einer Mess-, Steuer- und Bildaufzeichnungsvorrichtung verbunden. Diese Steuervorrichtung gewährleistet die der Augenausrichtung entsprechende Ausrichtung und/oder die der gemessenen Augenbrechkraft entsprechende Fokussierung der Kameras 3. Zudem bearbeitet und/oder speichert die Steuervorrichtung die von den Sensoren 4 und den Kameras 3 gelieferten Signale.

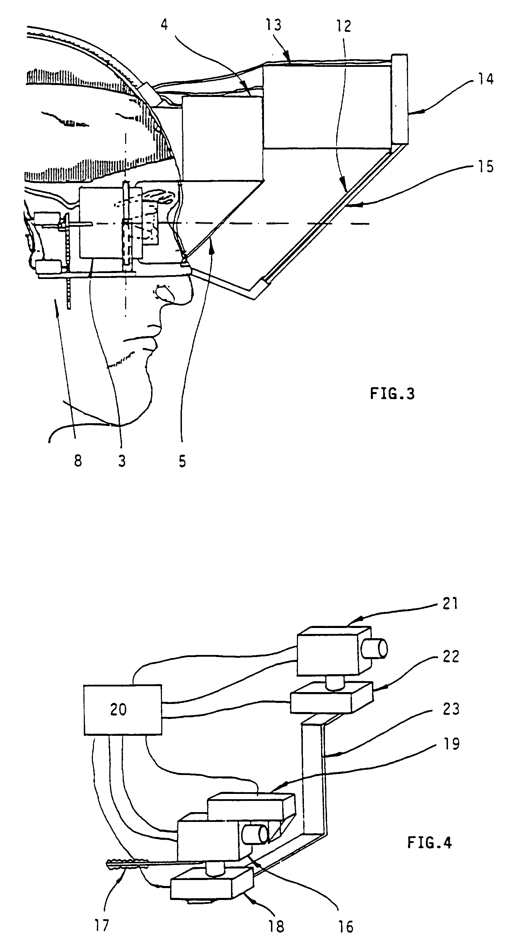

Fig.3 zeigt eine Ausführungsform, bei der nebst dem Messen von Augenparametern und der Bildaufnahme mittels der entsprechend der gemessenen Parameter eingestellten Kameras 3 gegebenenfalls auch vorgesehen ist, dass mindestens einem Auge über ein teildurchlässiges zweites optisches Umlenkelement 12 Bilder von einer mit der Halterung 6 verbundenen Bildquelle 13 zugeführt werden können. Um die Bilder sowohl dem Hintergrund überlagert als auch ausschliesslich dem Auge zuführbar zu machen, wird gegebenenfalls - vom Auge 2 aus gesehen - hinter dem die Bilder zuführenden Umlenkelement 12 ein von einer elektronischen Einheit 14 schaltbares Schliesselement 15 vorgesehen, das z.B. aufgrund von Flüssigkristalländerungen von einem transmissiven in einen reflektiven Zustand übergeht. Die Steuerung der Bildquelle 13 und der Einheit 14 erfolgt durch die Steuervorrichtung.

Fig.4 zeigt eine Ausführungsform, bei der das erste die Bildinformationen aufnehmende Sensorsystem und auch das Kontrollsystem je mindestens eine Videokamera umfasst. Anstatt die von einem Auge erfassten Bilder erfassbar zu machen, soll das Kontrollsystem parallel zu einer ersten Videokamera Bilder erfassen. Eine erste Videokamera 16 wird dabei etwa über einen Führungsgriff 17 manuell bewegt. Zur Bestimmung der Ausrichtung der ersten Kamera 16 ist dieser eine Richtungsbestimmungseinheit 18 zugeordnet. Die jeweilige Einstellung der Optik der ersten Kamera wird gegebenenfalls entweder direkt durch von der Kamera 16 abgegebene Signale der Optikstelleinrichtung oder durch eine an der Kamera 16 befestigte Messvorrichtung 19 bestimmbar gemacht.

Die erfassten Parameter der ersten Kamera 16 werden einer Steuervorrichtung 20 zugeführt, die entsprechend der gemessenen Parameter eine zweite Kamera 21 bzw. deren Ausrichtung und/oder die Einstellung ihrer Optik steuert. Die Ausrichtung erfolgt dabei etwa durch eine Ausrichtungsvorrichtung 22, auf der die zweite Kamera 21 befestigt ist. Gegebenenfalls sind die Halterungen 18 und 22 der beiden Kameras 16 und 21 über eine Haltevorrichtung 23 miteinander verbunden.

Darüber hinaus liegen im Rahmen der Erfindung aber auch Anwendungen, bei denen das Kontrollsystem absichtlich andere Parameter als das Sensorsystem aufweist; z.B. könnte das Sensorsystem (z.B. Videokamera eines Roboters) im Telezoombereich arbeiten, während das Kontrollsystem im Weitwinkelbereich eine Übersichtsfunktion ausübt. So kann es weiters von Vorteil sein, dass das Kontrollsystem in einem bestimmten Lichtwellenbereich parallel zum Sensorsystem arbeitet, wobei dieses in einem anderen Lichtwellenlängenbereich arbeitet.

## Patentansprüche

1. Verfahren zum Erfassen von Bildinformationen über die Informationsaufnahme eines optische Informationen aufnehmenden stereoskopischen Sensorsystems mit einem parallel zum Sensorsystem (2,16) angeordneten bildaufnehmenden Kontrollsystem (3,21), dessen Informationsaufnahme mit der Informationsaufnahme des Sensorsystems (2,16) so wenigstens annähernd zur Deckung gebracht wird, dass wenigstens ein optischer Parameter des Sensorsystems (2,16) - vorzugsweise laufend oder intermittierend - erfasst wird und der entsprechende optische Parameter des Kontrollsystems (3,21) in Abhängigkeit davon angepasst wird, **dadurch gekennzeichnet, dass** aus der Stereobasis des Sensorsystems (2,16) - im Falle von Augen aus dem Augenabstand - und den Ausrichtungen der beiden stereoskopischen Sensorteile des Sensorsystems (2,16) ― im Falle von Augen der Ausrichtungen der Augen (2) - ein Fixierpunkt bestimmt wird, auf den das bildaufnehmende Kontrollsystem (3,21) ― im Falle von Kameras mindestens eine Kamera (3), vorzugsweise zwei Kameras (3) - ausgerichtet wird bzw. werden.

2. Verfahren nach Anspruch 1, **dadurch gekennzeichnet, dass** bei der Bestimmung der Ausrichtung der beiden stereoskopischen Sensorteile des Sensorsystems (2, 16) die durch mindestens eine Optik definierte mindestens eine optische Achse erfasst wird.

3. Verfahren nach Anspruch 2, **dadurch gekennzeichnet, dass** die Bestimmung der Ausrichtungen der beiden stereoskopischen Teile des Sensorsystems (2,16) mittels Sensoren (4) erfolgt, das Kontrollsystem mit dem Kopf eines Anwenders verbunden wird und die bestimmten Richtungen zum Ausrichten mindestens einer, vorzugsweise aber zweier Kameras (3) verwendet werden.

4. Verfahren nach Anspruch 3, **dadurch gekennzeichnet, dass** zusätzlich mindestens einer der folgenden Verfahrensschritte vorgesehen ist:
a) bei der Bestimmung der Ausrichtung eines Auges (2) wird ein Bild des Auges (2) vorzugsweise im sichtbaren Lichtbereich oder gegebenenfalls im Infrarotbereich über ein vor dem Auge (2) angeordnetes, vorzugsweise zumindest für sichtbares Licht teildurchlässiges erstes Umlenkelement (5) aus dem zentralen Bereich des Gesichtsfeldes ausgekoppelt und einer Augenbildaufnahmevorrichtung (4) - vorzugsweise einem CCD - zugeführt, wobei zum Bestimmen der optischen Achse aus dem Bild die Pupillenlage und insbesondere das Zentrum der Pupille bestimmt wird;
b) um die Lage der Augen (2) bzw. die Lage der Pupillenzentren bei gerade nach vome gerichteten Augen (2), insbesondere den Augenabstand und/oder die Lage der Augendrehzentren, zu bestimmen, wird ein Justierschritt durchgeführt, bei dem die Augen vorzugsweise auf mit dem Kopf verbundene Markierpunkte ausgerichtet werden;
c) um der Augenausrichtung eine Achse zuzuordnen, wird die optische Augenachse durch das Augendrehzentrum und durch das aktuelle Pupillenzentrum gelegt;
d) die Gesichtslinie bzw. Fixierlinie jedes Auges (2) wird mittels einer Winkelkorrektur aus der Augenausrichtung bestimmt, wobei für die Winkelkorrektur gegebenenfalls eine Standardkorrektur, insbesondere aber eine Korrektur, die durch eine Winkelbestimmung beim Fixieren mindestens eines vorgegebenen Fixpunktes bestimmbar ist, verwendet wird.

5. Verfahren nach einem der vorangehenden Ansprüche, **dadurch gekennzeichnet, dass** zusätzlich die Brechkraft mindestens eines Auges (2) mittels Augenrefraktometrie bestimmt wird, wobei im wesentlichen die sphärische Dioptrie und gegebenenfalls der Astigmatismus und die Astigmatismusachse bestimmt werden.

6. Verfahren nach einem der vorangehenden Ansprüche, **dadurch gekennzeichnet, dass** zur Einstellung des Fokus mindestens einer Kamera (3) des Kontrollsystems mindestens einer der folgenden Verfahrensschritte vorgesehen ist:
a) der Fokus wird auf die Distanz zwischen der Kamera (3) und dem von den Augen (2) fixierten Fixierpunkt eingestellt;
b) der Fokus wird von der auf den Fixierpunkt ausgerichteten Kamera (3) automatisch eingestellt;
c) der Fokus wird in Abhängigkeit von der an mindestens einem Auge (2) gemessenen Brechkraft eingestellt.

7. Verfahren nach einem der Ansprüche 2 bis 6, **dadurch gekennzeichnet, dass** die Bildinformation des Kontrollsystems und gegebenenfalls die am ersten bildaufnehmenden System gemessenen Parameter aufgezeichnet werden.

8. Verfahren nach einem der Ansprüche 3 bis 7, **dadurch gekennzeichnet, dass** mindestens einem Auge (2) über ein teildurchlässiges zweites optisches Umlenkelement (12) Bilder zugeführt werden, wobei es sich bei diesen Bildern etwa um Bilder aus dem Kontrollsystem oder um Bilder von anderen bildgebenden Verfahren - z.B. Ultraschall- Röntgen-, CT-, PETund vergleichbare Analyseverfahren - handelt.

9. Bildaufnahmevorrichtung zur Erfassung einer Informationsaufnahme eines ersten stereoskopischen Sensorsystems (2,16) nach einem der vorhergehenden Verfahrensansprüche, wobei parallel zum Sensorsystem (2,16) ein Kontrollsystem (3,21) mit mindestens einer Videokamera (3,21) vorgesehen ist, deren optische Parameter den optischen Parametern des Sensorsystems (2,16) wenigstens annähernd angepasst sind, wobei das Kontrollsystem eine Nachführvorrichtung (8,22) umfasst, die im Betriebszustand Bewegungen oder Änderungen wenigstens eines optischen Parameters des Sensorsystems (2,16) erfasst und im Betriebszustand Einstellungen mindestens eines Parameters des Kontrollsystems kongruent nachführt, **dadurch gekennzeichnet, dass** das Kontrollsystem Mittel zur Ermittlung des Fixierpunkts der beiden stereoskopischen Teile des Sensorsystems aus deren Stereobasis und Ausrichtung umfaßt, sowie Mittel zum Einstellen des Kontrollsystems auf diesen Fixierpunkt.

10. Bildaufnahmevorrichtung nach Anspruch 9, **dadurch gekennzeichnet, dass** mindestens das Kontrollsystem eine Messvorrichtung (4) umfasst, die einen Parameter des Sensorsystems (2,16) bestimmbar macht und mindestens ein Steuerparameter des bildaufnehmenden Kontrollsystems (3,21) abhängig vom gemessenen Parameter einstellbar ist.

11. Bildaufnahmevorrichtung nach Anspruch 10, **dadurch gekennzeichnet, dass** die Messvorrichtung (4)
a) einen Achs-Detektor für mindestens eine optische Achse des Sensorsystems (2,16) und/oder
b) mindestens einen Brechkraftdetektor
für die Ermittlung der Fokuseinstellung einer Optik des Sensorsystems (2,16) umfasst.

12. Bildaufnahmevorrichtung nach einem der Ansprüche 9 bis 11, **dadurch gekennzeichnet, dass** die Nachführvorrichtung (8,22) so adaptiert ist, dass
a) mindestens eine optische Achse des Kontrollsystems (3,21), insbesondere die Ausrichtung mindestens einer Videokamera (3,21), und/oder
b) mindestens eine Fokuseinstellung einer Optik des Kontrollsystems (3,21), insbesondere der Fokus einer Videokamera, nachstellbar ist.

## Claims

1. Process for collecting image information from an information record of a stereoscopic sensor system recording optical information having an image-recording control System (3,21) in parallel with the sensor system (2,16), the information record of which is made to coincide at least approximately with the information record from the sensor system (2,16) such that at least one optical parameter of the sensor system (2,16) is determined, preferably continuously or intermittently, and the corresponding optical parameter of the control system (3,21) is varied in dependence on it, **characterized by** the fact that a fixation point is determined from the stereo base of the sensor system (2,16) - from the interpupillary distance, in the case of the eyes - and the directions of the two stereoscopic sensor parts of the sensor system (2,16) - the directions of the eyes (2), in the case of the eyes - onto which the image-recording control system (3,21) - at least one camera (3) in the case of cameras, but preferably two cameras (3) is or are aimed.

2. Process according to Claim 1, **characterized by** the fact that at least one optical axis is determined by means of at least one optical system in the determination of the direction of the two stereoscopic sensor parts of the sensor system (2,16).

3. Process according to Claim 2, **characterized by** the fact that the determination of the directions of the two stereoscopic parts of the sensor system (2,16) is accomplished by means of sensors (4), the control system is linked with the head of a user, and the directions determined are used to aim at least one, but preferably two cameras (3).

4. Process according t o Claim 3, **characterized by** the fact that a t least one of the following process steps is provided in addition:
a. in the determination of the direction of an eye (2) an image of the eye (2), preferably in the visible light range, or if necessary in the infrared range, is coupled out of the field of view by means of an initial deflecting element(5) placed in front of the eye (2), preferably partially transparent at least for visible light, and directed to a means (4) for recording the image of the eye, preferably a CCD, by means of which the position of the pupil and, in particular, the position of the center of the pupil, is determined in order to determine the optical axis from the image;
b. an adjustment step in which the eyes are preferably directed at a marking point linked to the head is carried out to determine the position of the eyes (2), or the positions of the centers of the pupils with the eyes (2) directed straight ahead;
c. the visual axis is established through the center of rotation of the eye and through the current center of the pupil to specify one visual axis;
d. the line of sight or fixation line of each eye(2) is determined from the eye direction by means of an angular correction, with a standard correction used for the angular correction if necessary, but in part fixation correction is used.

5. Process according to one of the foregoing Claims, **characterized by** the fact that refractive power of the eye (2) is determined by eye refractometrie wherein essentially the spherical dioptric power, the astigmatism, and the astigmatism axis are determined.

6. Process according to one of the foregoing Claims, **characterized by** the fact that at least one of the following process steps is provided to adjust the focus of at least one camera (3) of the control system:
a. the focus is adjusted to the distance between the camera (3) and the fixation point upon which the eye (2) is fixated;
b. the focus of the camera (3) directed toward the fixation point is adjusted automatically; and
c. the focus is adjusted, depending on the refractive power measured from at least one eye.

7. Process according to one of the Claims 2 to 6, **characterized by** the fact that the image information from the control system and if necessary the parameters measured at the initial image-recording system are recorded.

8. Process according to one of the Claims 3 to 7, **characterized by** the fact that images are directed to at least one eye (2) through a partially transparent second optical deflecting element (12), these images being images from the control system or images from other imaging processes (e. g., ultrasound, X-ray, CT-, PET-, and comparable analytical processes).

9. Image-recording system for collecting information from a first sensor system (2,16), according to one of the foregoing process Claims, in which a control system having at least one video camera (3,21), the optical parameters of which are at least approximately matched to the optical parameters of the sensor system, is provided in parallel with the sensor system (2,16) such that the control system includes a tracking means (8, 22) which in the operating condition registers movements or changes of at least one optical parameter of the sensor system (2,16) and in the operating condition congruently tracks adjustments of at least one parameter of the control system, **characterized by** the fact that the control system includes a means for determining the fixation point of the two stereoscopic parts of the sensor system form their stereo base and directions, and means for adjusting the control system to the fixation point.

10. Image-recording system according to Claim 9, **characterized by** the fact that at least one measuring means (4) is provided, which makes it possible to determine a parameter of the sensor system (2,16) and at least one control parameter of the image-recording control system (3,21) is adjustable depending on the measured parameter.

11. Image-recording System according to Claim 10 **characterized by** the fact that the measuring means (4) includes
a. at least one axis detector for at least one optical axis of the sensor system (2,16) and/or
b. at least one refractive power detector for determination of the focal adjustment of an 5 optical unit of the sensor system (2,16).

12. Image-recording System according to one of the claims 9 to 11, **characterized by** the fact that the tracking means (8, 22) is adapted so that:
a. at least one optical axis of the control system (3,21), especially the direction of at least one video camera (3,21), and/or
b. at least one focal adjustment of an optical unit of the control system (3,21), especially the focus of a video camera, is adjustable.

## Revendications

1. Méthode à saisir des informations d'image un enregistrement d'information d'un système de détecteurs stéréoscopique enregistrant des informations optiques avec un système de contrôle ordonné parallèlement au système de détecteurs (2,16) enregistrant des images, dont l'enregistrement d'information est apporte à la couverture au moins approximativement avec l'enregistrement d'information du système de détecteurs (2,16), de sorte que au moins un paramètre optique du système de détecteurs (2,16) - de préférence courant ou intermittent - est saisi et de sorte que le paramètre optique correspondant du système de contrôle (3,21) est adapté en dépendance de cela, **caractérisé en ce que,** de la base stéréo du système de détecteurs (2,16) - en cas de yeux de la distance d'oeil - et de l'orientation des deux parts de détecteur stéréoscopiques du système de détecteurs (2,16) - en cas de yeux des alignements des yeux (2) - un point de fixer est détermine, sur lequel le système de contrôle (3,21) enregistrant d'image - en cas de caméras au moins une caméra (3), mais deux caméras (3) de préférence - est aligné ou sont alignés.

2. Méthode selon revendication 1, **caractérisé en ce que**, à la détermination de l'orientation des deux parts de détecteur stéréoscopiques du système de détecteurs (2, 16) au moins une axe optique défini par au moins une optique est saisi.

3. Méthode selon revendication 2, **caractérisé en ce que**, la détermination de l'alignement des deux parts de détecteur stéréoscopiques du système de détecteurs (2, 16) est réalisé par des détecteurs (4), le système de contrôle est relié à la tête d'un utilisateur et les directions déterminées sont utilisées pour l'alignement de au moins une, de préférence deux caméras (3).

4. Méthode selon revendication 3, **caractérisé en ce que**, au moins un des pas de procédé suivants est prévu :
a) à la détermination de l'alignement d'oeil un image d'oeil, de préférence dans la gamme de lumière visible ou dans la gamme infrarouge, est débranché de la région central du champ visuel par un élément de déflection (5) ordonné devant l'oeil (2), qui est de préférence transparent pour de la lumière visible, et est alimenté à une dispositif d'enregistrement d'image d'oeil (4) -de préférence CCD -, cependant la position de pupille et en particulier le centre de la pupille est déterminé pour déterminer l'axe optique.
b) pour déterminer la position des yeux (2), ou respectivement les positions des centres de pupille quand les yeux sont dirigés vers l'avance, en particulier la distance d'oeil et/ou la position des centres de tourner l'oeil, un pas d'ajuster est effectué, dont les yeux sont orientés vers les points de marquer de préférence reliés à la tête.
c) pour attribuer un axe à l'alignement d'oeil, l'axe d'oeil optique est mis à travers du centre de tourner l'oeil et à travers le centre de pupille actuel.
d) la ligne visuel ou respectivement la ligne de fixer de chaque oeil (2) est déterminé de l'alignement d'oeil au moyen de correction d'angle, cependant, le cas échéant, une correction standard est utilisé, mais en particulier une correction, qui est déterminable par une détermination d'angle pendant fixer de au moins un point fixe.

5. Méthode selon l'une quelconque des revendications précédentes, **caractérisé en ce que**, la puissance d'au moins un oeil (2) est déterminée au moyen de la réfractométrie d'oeil en déterminant essentiellement la dioptrie sphérique et le cas échéant l'astigmatisme et l'axe d'astigmatisme.

6. Méthode selon l'une quelconque des revendications précédentes, **caractérisé en ce que**, au moins un des pas de procédé suivants est prévu pour le réglage du foyer d'au moins une caméra (3) du système de contrôle:
a) le foyer est réglé sur la distance entre la caméra (3) et le point de fixer fixé par les yeux (2) ;
b) le foyer est réglé automatiquement par la caméra (3) orientée vers le point de fixer ;
c) le foyer est réglé en dépendance de la puissance mesurée au moins à un oeil (2).

7. Méthode selon l'une quelconque des revendications 2 à 6, **caractérisé en ce que**, l'information d'image du système de contrôle et le cas échéant les paramètres mesurés au premier système qui enregistre l'image sont enregistrés.

8. Méthode selon l'une quelconque des revendications 3 à 7, **caractérisé en ce que**, au moins un oeil (2) est alimenté en images par un deuxième dispositif de déflection optique (12) part perméable, et qu'il s'agit d'images du système de contrôle ou d'images d'autres méthodes qui donnent l'image par ex. méthodes d'ultrason, de radiographie, CT, PET et des méthodes d'analyse et comparables.

9. Dispositif d'enregistrement d'image à l'enregistrement d'information d'un premier système de détecteurs (2,16) stéréoscopique selon l'une quelconque des revendications précédentes, dont parallèle au système de détecteurs (2,16) un système de contrôle (3,21) est prévu avec au moins une caméra vidéo (3,21, dont ses paramètres optiques sont attribués au moins approximativement aux paramètres optiques du système de détecteurs (2,16) et dont le système de contrôle comprend un dispositif de guidage (8,22), qui dans l'état de fonctionnement saisit des mouvements ou des changements de au moins un paramètre optique du système de détecteurs (2,16) et qui dans l'état de fonctionnement poursuite des réglages d'au moins un paramètre du système de contrôle congrument, **caractérisé en ce que**, le système de contrôle comprend un dispositif de fixer pour la distance du base stéréo des deux parts du système de détecteurs stéréoscopique et pour son alignement.

10. Dispositif d'enregistrement d'image selon revendication 9, **caractérisé en ce que**, au moins le système de contrôle comprend un dispositif de mesure (4), qui fait déterminable l'un des paramètres du système de détecteurs (2,16), et que au moins un paramètre de commande du système de contrôle (3,21) enregistrant l'image est mesuré dépendamment du paramètre réglable.

11. Dispositif d'enregistrement d'image selon revendication 10, **caractérisé en ce que**, le dispositif de mesure (4) comprend:
a) un détecteur d'axe pour au moins un axe optique du système de détecteurs (2,16) et/ou
b) au moins un détecteur de puissance convergent pour la détermination de l'ajustage du foyer d'une optique du système de détecteurs (2,16).

12. Dispositif d'enregistrement d'image selon l'une quelconque des revendications 9 à 11, **caractérisé en ce que**, le dispositif de guidage (8,22) est adapté de sorte que,
a) au moins un axe optique du système de contrôle (3,21), en particulier l'alignement d'au moins une caméra vidéo (3,21), et/ou
b) au moins un réglage de foyer d'une optique du système de contrôle (3,21), en particulier le foyer d'une caméra vidéo,
est réglable poursuivrement.
